# EUROPÄISCHE PATENTSCHRIFT

(11) **EP 0 655 231 B1**
(45) Veröffentlichungstag und Bekanntmachung des Hinweises auf die Patenterteilung: **03.11.1999**
(21) Anmeldenummer: 94250198.2
(22) Anmeldetag: 12.08.1994
(51) Int. Cl.: A61F 5/01, A61G 7/07

(54) **Orthopädische Kopf- bzw. Nackenstütze (Cervikalextensor)**
Orthopaedic head or neck support (cervical extensor)
Soutien orthopédique du cou ou de la tête (extenseur cervical)

(30) Priorität: 17.08.1993 DE 4328335
(43) Veröffentlichungstag der Anmeldung: 31.05.1995
(73) Patentinhaber: Toth-Kischkat, Anna, Dipl.-Phys., 12163 Berlin (DE)
(72) Erfinder: Toth-Kischkat, Anna, Dipl.-Phys., 12163 Berlin (DE)
(74) Vertreter: Meissner, Peter E., Dipl.-Ing.

(56) Entgegenhaltungen:
- DE-A- 3 302 078
- US-A- 2 789 625
- US-A- 5 147 287

## Beschreibung

Die Erfindung betrifft eine orthopädische Kopf- bzw. Nackenstütze.
Beschwerden, wie beispielsweise Nackenhinterkopfschmerzen, lokalisiert oder ausstrahlend in die Arme, Schwindel, Gleichgewichtsstörungen, Übelkeit, Ohrensausen, Hörsturz, Augensymptome z. B. Einengung des Gesichtsfeldes, weiße Blitze vor den Augen, psychosensorische Symptome z. B. Wattegefühl, Distanzgefühl, die Welt wird durch eine Milchglasscheibe gesehen, Störungen des Mittelzeitgedächtnisses, sind Symptome folgender Erkrankungen an der Halswirbelsäule: Migräne, sogenannte Occipitalis Neuralgie, Blockierungen im chirotherapeutischen Sinne der Halswirbelsäule (HWS), Zustand nach HWS Distorsionen (HWS Schleudertrauma), Verkürzungen der Nackenmuskulatur mit dabei auftretenden Beschwerden sei es haltungsbedingt am Arbeitsplatz oder verursacht durch die Form der Halswirbelsäule, Muskeldysbalancen durch Seitabweichungen der HWS (Skoliose).

Behandlungsmöglichkeiten, die versuchen ursächlich einzuwirken, sind:
- Krankengymnastik mit Dehnung und Kräftigung der Muskulatur
- Chirotherapeutische Griffe mit Mobilisation und Manipulation der Gelenke,
- eventuell verschiedene Techniken der Massage,
- verschiedene Injektionsbehandlungen dort,
- Medikamente,
- Bestrahlungen.
Diese Behandlungsmethoden sind teilweise aufwendig und können bis auf wenige vom Patienten nicht selbsttätig durchgeführt werden.

Zur Dehnungsbehandlung der HWS Muskulatur und der beteiligten Gelenke wird von Ärzten, Krankengymnasten und Masseuren der sogenannte Gabelgriff angewendet. Dabei wird aus abgespreiztem Daumen und gestrecktem Zeigefinger eine Gabel gebildet, welche am Hinterkopf angelegt den Kopf gegenüber der HWS zieht. Dieser Griff ist für den Anwender nach kurzer Zeit ermüdend.

Nachteile bisheriger Behandlungsmethoden sind u. a. Beeinträchtigung der Lebensführung durch häufige Arztbesuche, schmerzhafte Behandlungen (Injektionen), unerwünschte Medikamentennebenwirkungen, Schmerzsymtome, die auftreten zu Zeiten, in denen der Behandler nicht ohne weiteres erreichbar ist, können nicht gelindert werden.

Aus der US 2.789.625 ist eine Kopf- und Nackenstütze bekannt, die zwei im Abstand nebeneinander fest angeordnete U-förmige Stützen aufweist, zwischen denen die Auflage für den Kopf oder Nacken einer Person angeordnet ist. Die Stützen sind höhenverstellbar. Diese Einrichtung dient als Auflage bei Kopfwäschen, im klinischen Bereich oder Dentalbereich. Ferner ist aus der DE 33 02 078 A1 ein Wirbelsäulengerät bekannt, das auf den Oberkörper eines Patienten aufsetzbar ist und zur Abstützung des Kopfes dient.

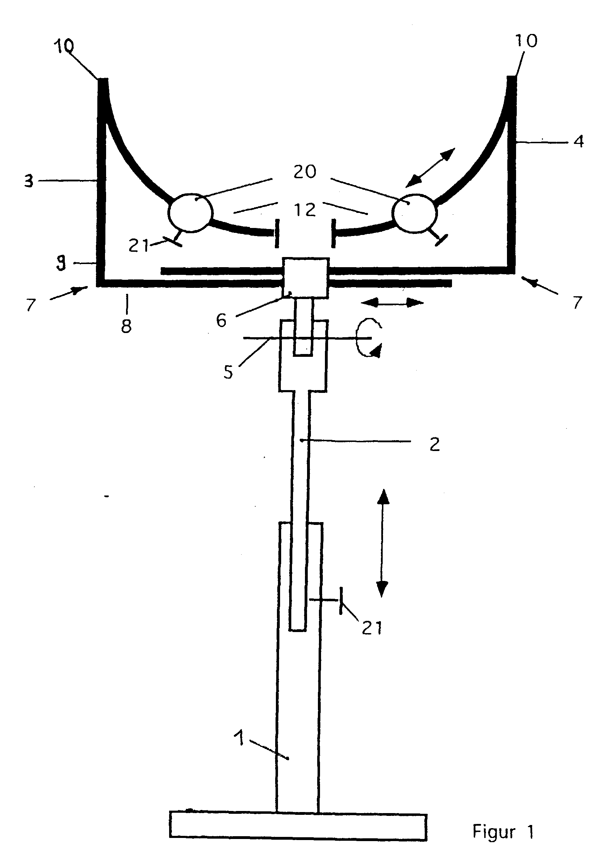

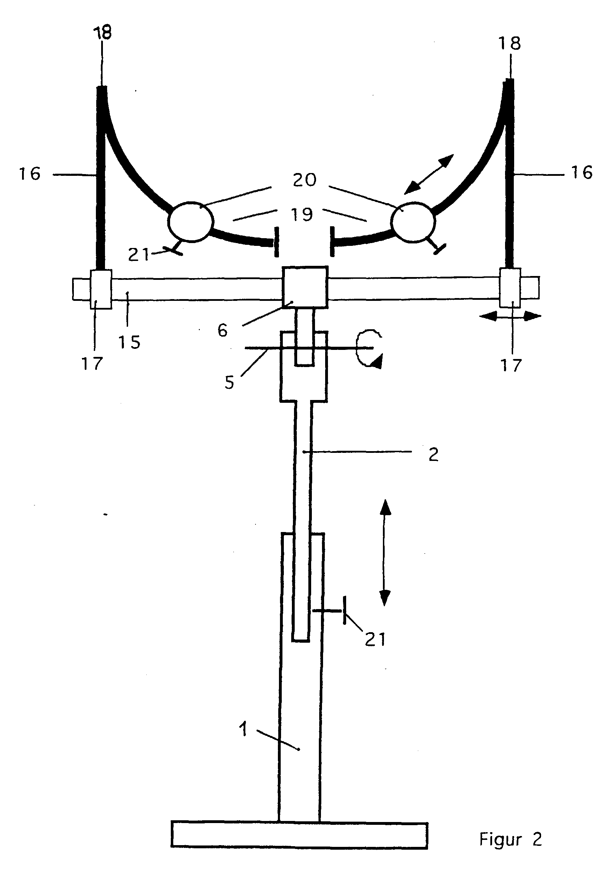

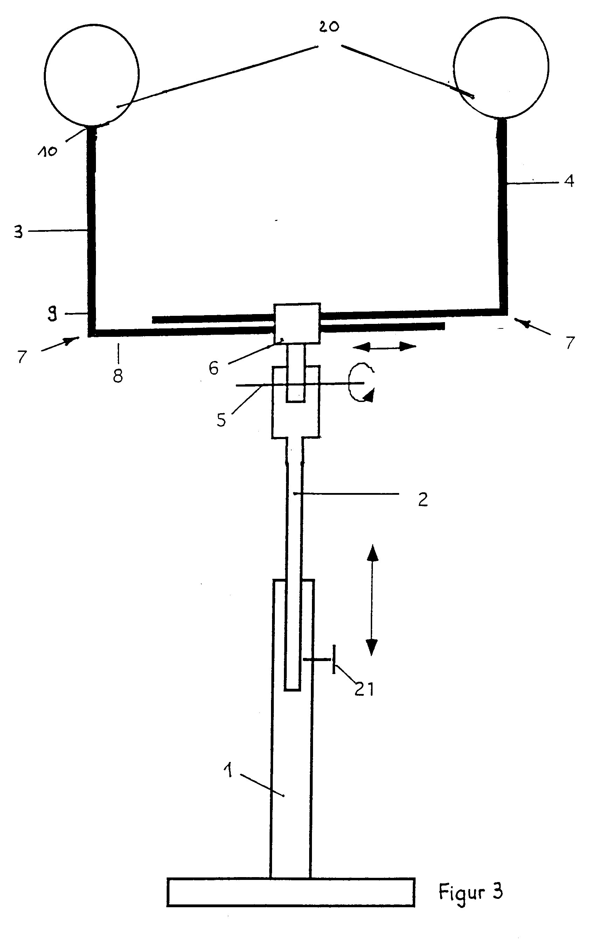

Der Erfindung liegt die Aufgabe zugrunde, eine orthopädische Kopf- bzw. Nackenstütze zu schaffen, die nach ärztlicher Indikation auch vom Patienten allein z. B. zu Hause angewandt werden kann.
Gelöst wird diese Aufgabe mit den Merkmalen im Anspruch 1 bzw. 6. Vorzugsweise Ausgestaltungen ergeben sich aus den übrigen Unteransprüchen.
Die Erfindung soll anhand von drei speziellen Ausführungsbeispielen erläutert werden. Den in den Figuren 1 bis 3 dargestellten Ausführungsbeispielen ist gemeinsam, daß die Kopf- bzw. Nackenstütze aus einer Vertikalstütze 2 und einer Halterung 6 besteht. Wie sich aus den Zeichnungen ergibt, ist die Vertikalstütze 2 in der Höhe veränderbar, wobei die Arretierung durch das Teil 21, das symbolisch angedeutet ist, erfolgt. Am oberen Ende der Vertikalstütze 2 befindet sich eine horizontale Achse 5. Diese Achse nimmt die Halterung 6 auf. Die Halterung 6 dient zur Aufnahme der Auflagenteile 3 und 4 bzw. der Abstandsteile 16. Nach den Ausführungen in den Figuren 1 und 3 sind zwei L-förmige Stützteile 7 vorgesehen die jeweils einen längeren Schenkel 8 und einen kürzeren Schenkel 9 aufweisen. Die beiden längeren Schenke 8 werden von der Halterung 6 aufgenommen, und es kann in der Halterung bzw. an der Halterung je nach der Aufnahme dieser Schenkel 8 eine relative Verschiebung der beiden Stützteile 7 zueinander erfolgen, wobei auch hier eine Arretierung vorgesehen ist. Auf diese Weise läßt sich der Abstand der freien Enden der kürzeren Schenkel 9, die mit 10 bezeichnet sind, verändern. Bei dem in Figur 1 dargestellten Beispiel befinden sich an den freien Enden 10 noch Ansätze 12, die darauf verschiebliche kugelförmige Auflagen 20 aufnehmen. Auch diese sind arretierbar, wie es mit 21 angedeutet ist. Diese kugelförmigen Auflagen 20 dienen zur Abstützung des Nacken- bzw. Kopfbereiches des Patienten. In der Ausführung nach Fig. 3 sind die Ansätze 12 weggelassen und die Auflagen 20 direkt an den freien Enden 10 der Schenkel 9 angebracht. Die Auflagen 20 können punktförmig oder flächenförmig sein, je nach gewünschter orthopädischer Wirkung.
Bei der Ausführung nach Figur 2 sind die L-förmigen Stützteile durch eine Schiene 15 ersetzt, auf der Schlitten 17 verschiebbar angeordnet sind, und diese Schlitten 17 tragen Abstandsteile 16. Hier sind die freien Enden mit 18 bezeichnet bzw. die Ansätze mit 19, so daß diesbezüglich Übereinstimmung zu der Ausführung nach Figur 1 besteht. Auch hier müssen die Ansätze 19 nicht unbedingt vorhanden sein, sondern die freien Enden 18 können, wie in der Ausführung nach Figur 3 mit Auflagen versehen sein.
Für die Behandlung wird der Patent auf eine horizontale Unterlage mit dem Rücken gelegt, und die erfindungsgemäße Kopf- bzw. Nackenstütze wird hinter bzw. unterhalb seines Kopfes so angeordnet, daß der Nackenbereich bzw. Kopfbereich von den kugelförmigen Auflagen 20 aufgenommen wird, und zwar so, daß eine gewisse Streckung des oberen Bereiches des Halswirbels erfolgt. Erreicht wird damit eine Imitation des sogenannten Gabelgriffes, ohne die vorher erwähnten Nachteile. Der Einsatz des Gerätes ist nebenwirkungsfrei, da nur das Eigengewicht der HWS und frei hängender Teile des Schultergürtels ziehen, während der Kopf des Patienten das Gegengewicht darstellt. Damit kann der Patient - nach vorgegebener Indikation - die Behandlung jederzeit auch zu Hause selbst durchführen.

## Patentansprüche

1. Orthopädische Kopf- bzw. Nackenstütze mit einer Vertikalstütze (2) sowie zwei daran angeordneten punkt- oder flächenartig ausgebildeten, im horizontalen Abstand voneinander vorgesehenen Auflagen für den Kopf bzw. Nacken des Patienten, gekennzeichnet durch folgende Merkmale:
a) am oberen Ende der auf einer Fußplatte (1) befestigbaren und höhenverstellbaren Vertikalstütze (2) ist eine horizontale Achse (5) vorgesehen,
b) um diese Achse (5) ist eine Halterung (6) schwenkbar,
c) von der Halterung (6) wird jeweils der längere Schenkel (8) zweier im wesentlichen L-förmiger Stützteile (7) aufgenommen, wobei diese Schenkel parallel zu der Achse (5) verlaufend gehalten sind,
d) die Stützteile (7) sind relativ zueinander in oder an der Halterung (6) verschiebbar angeordnet, so daß der Abstand der freien Enden der kürzeren Schenkel (9) voneinander veränderbar ist und
e) im Bereich der freien Enden der kürzeren Schenkel (9) sind die Auflagen für den Kopf bzw. Nacken des Patienten vorgesehen.

2. Orthopädische Kopf- bzw. Nackenstütze nach Anspruch 1,
dadurch gekennzeichnet
daß an den freien Enden (10) der kürzeren Schenkel (9) in Richtung auf die Halterung (6) weisende, jedoch im Abstand von dieser endende Ansätze (12) angeordnet sind.

3. Orthopädische Kopf- bzw. Nackensütze nach Anspruch 2,
dadurch gekennzeichnet,
daß auf den Ansätzen (12) jeweils eine kugelförmige Auflage (20) für den Kopf bzw. Nacken des Patienten verschiebbar angeordnet ist.

4. Orthopädische Kopf- bzw. Nackenstütze nach Anspruch 3,
dadurch gekennzeichnet,
daß die Auflagen (20) auswechselbar befestigt sind.

5. Orthopädische Kopf- bzw. Nackenstütze nach Anspruch 1,
dadurch gekennzeichnet,
daß an den freien Enden (10) der kürzeren Schenkel (9) jeweils eine kugelförmige Auflage (20) direkt angebracht ist.

6. Orthopädische Kopf- bzw. Nackenstütze mit einer Vertikalstütze (2) sowie zwei daran angeordneten, punkt- oder flächenartig ausgebildeten, im horizontalen Abstand voneinander vorgesehenen Auflagen für den Kopf bzw. Nacken des Patienten, gekennzeichnet durch folgende Merkmale:
a) am oberen Ende der auf einer Fußplatte (1) befestigbaren und höhenverstellbaren Vertikalstütze (2) ist eine horizontale Achse (5) vorgesehen,
b) um die Achse (5) ist eine Halterung (6) schwenkbar,
c) in oder an der Halterung ist eine parallel zur Achse (5) verlaufende Schiene (15) gehalten,
d) auf der Schiene (15) sind Schlitten (17) relativ zueinander derart verschiebbar, daß deren Abstand voneinander veränderbar ist,
e) an den Schlitten sind die Auflagen für den Kopf bzw. Nacken des Patienten vorgesehen.

7. Orthopädische Kopf- bzw. Nackenstütze nach Anspruch 6,
dadurch gekennzeichnet,
daß an den Schlitten (17) Abstandsteile (16) angeordnet sind, daß an den freien Enden dieser Abstandsteile (16) in Richtung auf die Halterung (6) weisende, jedoch im Abstand von dieser endende Ansätze (19) angeordnet sind.

8. Orthopädische Kopf- bzw. Nackenstütze nach Anspruch 7,
dadurch gekennzeichnet,
daß auf den Ansätzen (19) jeweils eine kugelförmige Auflage (20) für den Kopf bzw. Nacken des Patienten verschiebbar angeordnet ist.

9. Orthopädische Kopf- bzw. Nackenstütze nach Anspruch 8,
dadurch gekennzeichnet,
daß die Auflage (20) auswechselbar befestigt sind.

10. Orthopädische Kopf- bzw. Nackenstütze nach Anspruch 6,
dadurch gekennzeichnet,
daß an den freien Enden (18) der Abstandsteile (16) jeweils eine kugelförmige Auflage (20) direkt angebracht ist.

## Claims

1. An orthopaedic head or neck support comprising a vertical support (2) and two point-like or surface-like rests spaced apart horizontally for the head or neck of the patient located thereon, characterised by the following features:
a) a horizontal spindle (5) is provided on the upper end of the vertically adjustable vertical support (2), which can be fastened to a footplate (1),
b) a holder (6) can be pivoted about this spindle (5),
c) the holder (6) in each case holds the longer arm (8) of two substantially L-shaped supporting parts (7), these arms being held extending parallel to the spindle (5),
d) the support parts (7) are arranged displaceably relative to one another in or on the holder (6), so that the distance between the free ends of the shorter arms (9) can be altered, and
e) the rests for the head or neck of the patient are provided in the region of the free ends of the shorter arms (9).

2. An orthopaedic head or neck support according to Claim 1, characterised in that attachments (12) which point towards the holder (6) but end spaced apart therefrom are arranged on the free ends (10) of the shorter arms (9).

3. An orthopaedic head or neck support according to Claim 2, characterised in that in each case a spherical rest (20) for the head or neck of the patient is displaceably arranged on the attachments (12).

4. An orthopaedic head or neck support according to Claim 3, characterised in that the rests (20) are fastened so as to be exchangeable.

5. An orthopaedic head or neck support according to Claim 1, characterised in that in each case a spherical rest (20) is attached directly to the free ends (10) of the shorter arms (9).

6. An orthopaedic head or neck support comprising a vertical support (2) and two point-like or surface-like rests located thereon spaced apart horizontally for the head or neck of the patient, characterised by the following features:
a) a horizontal spindle (5) is provided on the upper end of the vertically adjustable vertical support (2), which can be fastened to a footplate (1),
b) a holder (6) can be pivoted about the spindle (5),
c) a rail (15) extending parallel to the spindle (5) is held in or on the holder,
d) slides (17) are displaceable relative to each other on the rail (15) such that the distance between them can be altered,
e) the rests for the head or neck of the patient are provided on the slides.

7. An orthopaedic head or neck support according to Claim 6, characterised in that spacer parts (16) are arranged on the slides (17), that attachments (19) which point towards the holder (6) but end spaced apart therefrom are arranged on the free ends of these spacer parts (16).

8. An orthopaedic head or neck support according to Claim 7, characterised in that in each case a spherical rest (20) for the head or neck of the patient is displaceably arranged on the attachments (19).

9. An orthopaedic head or neck support according to Claim 8, characterised in that the rests (20) are fastened so as to be exchangeable.

10. An orthopaedic head or neck support according to Claim 6, characterised in that in each case a spherical rest (20) is attached directly to of the free ends (18) of the spacer parts (16).

## Revendications

1. Appui de tête ou de nuque orthopédique, comportant un appui vertical (2), ainsi que deux supports pour la tête ou la nuque du patient, qui y sont agencés, qui sont réalisés de façon ponctuelle ou superficielle et qui sont disposés avec un écartement horizontal entre eux, caractérisé par les caractéristiques suivantes :
a) à l'extrémité supérieure de l'appui vertical (2) pouvant être fixé sur un patin (1) et susceptible d'être réglé en hauteur, on prévoit un axe horizontal (5),
b) un élément de maintien (6) peut pivoter autour de cet axe (5),
c) l'élément de maintien (6) reçoit à chaque fois la branche (8) la plus longue de deux pièces d'appui (7) essentiellement en forme de L, ces branches étant maintenues de façon à s'étendre parallèlement à l'axe (5),
d) les pièces d'appui (7) sont agencées de façon à pouvoir être déplacées relativement l'une par rapport à l'autre dans ou sur l'élément de maintien (6) de sorte que l'écartement des extrémités libres des branches (9) les plus courtes peut être modifié, et
e) dans la zone des extrémités libres des branches (9) les plus courtes, on prévoit les supports pour la tête ou la nuque du patient.

2. Appui de tête ou de nuque orthopédique selon la revendication 1,
caractérisé en ce qu'aux extrémité libres (10) des branches (9) les plus courtes sont agencées des rallonges (12) s'étendant en direction de l'élément de maintien (6), mais se terminant de façon écartée de celui-ci.

3. Appui de tête ou de nuque orthopédique selon la revendication 2,
caractérisé en ce que sur les rallonges (12) est agencé, à chaque fois, de façon déplacable, un support sphérique (20) pour la tête ou la nuque du patient.

4. Appui de tête ou de nuque orthopédique selon la revendication 3,
caractérisé en ce que les supports (20) sont fixés de façon amovible.

5. Appui de tête ou de nuque orthopédique selon la revendication 1,
caractérisé en ce qu'aux extrémités libres (10) des branches (9) les plus courtes, est agencé, à chaque fois, directement un support sphérique (20).

6. Appui de tête ou de nuque orthopédique comportant un appui vertical (2), ainsi que deux supports pour la tête ou la nuque du patient, qui y sont agencés, qui sont réalisés de façon ponctuelle ou superficielle et qui sont disposés avec un écartement horizontal entre eux,
caractérisé par les caractéristiques suivantes :
a) à l'extrémité supérieure de l'appui vertical (2) pouvant être fixé sur un patin (1) et susceptible d'être réglé en hauteur, on prévoit un axe horizontal (5),
b) un élément de maintien (6) peut pivoter autour de cet axe (5),
c) dans ou sur l'élément de maintien est maintenu un rail (15) s'étendant parallèlement à l'axe (5),
d) des chariots (17) peuvent se déplacer l'un par rapport à l'autre sur le rail (15) de sorte que leur écartement peut être modifié,
e) sur les chariots, on prévoit les supports pour la tête ou la nuque du patient.

7. Appui de tête ou de nuque orthopédique selon la revendication 6,
caractérisé en ce que l'on agence sur les chariots (17) des pièces d'écartement (16), en ce qu'aux extrémités libres de ces pièces d'écartement (16) sont agencées des rallonges (19) s'étendant en direction de l'élément de maintien (6), mais se terminant de façon écartée de celui-ci.

8. Appui de tête ou de nuque orthopédique selon la revendication 7,
caractérisé en ce que sur les rallonges (19) est agencé, à chaque fois, de façon déplacable, un support sphérique (20) pour la tête ou la nuque du patient.

9. Appui de tête ou de nuque orthopédique selon la revendication 8,
caractérisé en ce que les supports (20) sont fixés de façon amovible.

10. Appui de tête ou de nuque orthopédique selon la revendication 6,
caractérisé en ce qu'aux extrémités libres (18) des pièces d'écartement (16), est agencé, à chaque fois, directement un support sphérique (20).
